(19) **Europäisches Patentamt / European Patent Office / Office européen des brevets**

(11) **EP 4 660 233 A1**

(12) **EUROPEAN PATENT APPLICATION**
published in accordance with Art. 153(4) EPC

(43) Date of publication:
**10.12.2025 Bulletin 2025/50**

(21) Application number: **24819593.5**

(22) Date of filing: **07.06.2024**

(51) International Patent Classification (IPC):
*C08K 5/1545* $^{(2006.01)}$     *C08K 5/17* $^{(2006.01)}$
*C08K 5/09* $^{(2006.01)}$     *C08K 5/053* $^{(2006.01)}$
*C08K 3/16* $^{(2006.01)}$     *C08J 3/24* $^{(2006.01)}$

(52) Cooperative Patent Classification (CPC):
C08J 3/24; C08K 3/16; C08K 5/053; C08K 5/09;
C08K 5/1545; C08K 5/17

(86) International application number:
**PCT/KR2024/007762**

(87) International publication number:
**WO 2024/253442 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.06.2023 KR 20230073035**
**05.06.2024 KR 20240073975**

(71) Applicant: **LG CHEM, LTD.**
**Seoul 07336 (KR)**

(72) Inventors:
- **MIN, Ji Hong**
  **Daejeon 34122 (KR)**
- **CHO, Hyemin**
  **Yuseong gu, Daejeon 34122 (KR)**

(74) Representative: **Plasseraud IP**
**104 Rue de Richelieu**
**CS92104**
**75080 Paris Cedex 02 (FR)**

(54) **COMPOSITION OF SUPER ABSORBENT POLYMER AND PREPARATION METHOD THEREOF**

(57) The present invention relates to a superabsorbent polymer composition and a preparation method thereof. Particularly, the present invention provides a superabsorbent polymer composition with deodorizing ability while minimizing deterioration in physical properties of the super absorbent polymer by controlling and mixing a deodorizing material combination, and a preparation method thereof.

**Description**

**[TECHNICAL FIELD]**

CROSS-REFERENCE TO RELATED APPLICATION

The present application is based on, and claims priority from, Korean Patent Application Nos. 10-2023-0073035 filed on June 7, 2023 and 10-2024-0073975 filed on June 5, 2024, respectively, the disclosures of which are hereby incorporated by reference herein in their entirety.

The present invention relates to a superabsorbent polymer composition and a preparation method thereof. Specifically, the present invention relates to a superabsorbent polymer composition with deodorizing ability and a preparation method thereof.

**[BACKGROUND ART]**

A superabsorbent polymer (SAP) is a synthetic polymeric material capable of absorbing moisture from 500 to 1000 times its own weight. Various manufacturers have denominated it as different names, such as SAM (Super Absorbency Material), AGM (Absorbent Gel Material), etc. Since such superabsorbent polymers started to be practically applied in sanitary products, and they are now being widely used not only for hygiene products such as disposable diapers for children, etc., but also for water retaining soil products for gardening, water stop materials for the civil engineering and construction, sheets for raising seedling, fresh-keeping agents for food distribution fields, materials for poultices, or the like.

In most cases, these superabsorbent polymers have been widely used in the field of sanitary materials such as diapers or sanitary napkins, etc. Inside the sanitary materials, the superabsorbent polymer is generally distributed throughout pulp. However, recent efforts have been continuously made to provide sanitary materials such as diapers having a thinner thickness, etc., and as part of that, diapers having a reduced content of pulp, and furthermore, diapers having no pulp, so-called pulpless diapers are actively under development.

As described above, such a sanitary material having a reduced content of pulp or having no pulp includes the superabsorbent polymer at a relatively high ratio, and the superabsorbent polymer particles are inevitably included as multiple layers in the sanitary materials. In order to allow the whole superabsorbent polymer particles included as multiple layers to more efficiently absorb liquid such as urine, etc., it is necessary that the superabsorbent polymer basically exhibits high absorption performance and high absorption rate. Further, the superabsorbent polymer should not release absorbed liquid even under an external pressure, and furthermore, requires liquid permeability to well retain its original shape even in a swollen state by absorbing liquid.

Accordingly, in order to improve the basic absorbency and water retention capacity of the superabsorbent polymer, much research is being conducted, such as surface crosslinking, etc.

Meanwhile, the superabsorbent polymer may be used in sanitary materials, and in this case, there may be a problem of reduced usability due to unpleasant odor of the absorbed liquid, such as human and pet excrement. In particular, there is a need to suppress both the odor initially contained in the absorbed liquid and the odor generated during the use of sanitary materials containing the superabsorbent polymer.

Traditionally, the superabsorbent polymer have been mixed with deodorizing materials. However, absorbency is lowered by mixing with an excessive amount of deodorizing materials in order to effectively suppress odor, or there has been a problem that the desired level of deodorizing ability could not be achieved only for antibacterial purposes.

Thus, there is a growing demand for unpleasant odor suppression, as well as for absorbency and water retention capacity, which are the basic physical properties of the superabsorbent polymer. Accordingly, it is necessary to prepare a superabsorbent polymer with excellent deodorizing ability.

**[DETAILED DESCRIPTION OF THE INVENTION]**

**[Technical Problem]**

There are provided a superabsorbent polymer composition with deodorizing ability and a preparation method thereof.

More specifically, there are provided a superabsorbent polymer composition with excellent deodorizing ability while minimizing deterioration of physical properties of the superabsorbent polymer by mixing with deodorizing materials by controlling a combination thereof, and a preparation method thereof.

**[Technical Solution]**

To achieve the above objects, there is provided a superabsorbent polymer composition as follows:

the superabsorbent polymer composition including
a superabsorbent polymer including a base polymer including a crosslinked polymer, in which an acrylic acid-based monomer having at least partially neutralized acidic groups and an internal crosslinking agent are crosslinking-polymerized, and a surface-crosslinked layer formed on the surface of the base polymer, in which the crosslinked polymer is additionally crosslinked via a surface crosslinking agent; and
tannic acid; an iodine compound; and a chelating agent.

There is also provided a method of preparing the superabsorbent polymer composition as follows:
the method including the steps of:

forming a water-containing gel polymer by crosslinking polymerizing an acrylic acid-based monomer having at least partially neutralized acidic groups in the presence of an internal crosslinking agent and a polymerization initiator (step 1);
preparing a base polymer including a crosslinked polymer by coarsely pulverizing, drying, and pulverizing the water-containing gel polymer (step 2);
preparing a mixture by mixing the base polymer with a surface crosslinking agent (step 3); and
preparing a superabsorbent polymer, in which a surface-crosslinked layer is formed on the surface of the base polymer, by heat treatment of the mixture (step 4);
wherein tannic acid and an iodine compound are mixed with the superabsorbent polymer of the step 4, in which the surface-crosslinked layer is formed, and a chelating agent is mixed in the coarse pulverization step of the step 2 or mixed with the superabsorbent polymer of the step 4, in which the surface-crosslinked layer is formed.

**[ADVANTAGEOUS EFFECTS]**

As described above, the present invention is characterized by providing a superabsorbent polymer composition with excellent deodorizing ability by applying deodorizing materials in combination to the superabsorbent polymer, and a preparation method thereof.
Specifically, the present invention is characterized by providing a superabsorbent polymer composition capable of removing both an unpleasant odor which is initially caused by urine discharged from the human body and an unpleasant odor which is caused by bacteria on the skin, etc. while using hygiene products or caused when the used hygiene products are left unattended, and a preparation method thereof.

**[DETAILED DESCRIPTION OF THE EMBODIMENTS]**

The terms used in this description are just for explaining exemplary embodiments and it is not intended to restrict the present invention. The singular expression may include the plural expression unless it is differently expressed contextually. It must be understood that the term "include", "equip", or "have" in the present description is only used for designating the existence of characteristics taken effect, steps, components, or combinations thereof, and do not exclude the existence or the possibility of addition of one or more different characteristics, steps, components, or combinations thereof beforehand.
The present invention may be variously modified and have various forms, and specific exemplary embodiments are exemplified and explained in detail in the following description. However, it is not intended to limit the present invention to the specific exemplary embodiments, and it must be understood that the present invention includes every modifications, equivalents, or replacements included in the spirit and technical scope of the present invention.
Hereinafter, according to specific embodiments of the present invention, a superabsorbent polymer composition and a preparation method thereof will be described in more detail.
Prior to this, the terminology used in this specification is only for referring to specific exemplary embodiments and is not intended to limit the present invention. Further, singular forms used herein include plural forms as well, unless the context clearly indicates otherwise.
For reference, the "superabsorbent polymer" in the present specification means the superabsorbent polymer itself, or it may be used to encompass those made to be appropriate for productization through additional processes, for example, surface crosslinking, fine powder reassembling, drying, pulverizing, classification, etc., according to the context.
Further, the "base polymer" or "base polymer powder" in the present specification means particles or powders made by drying and pulverizing a polymer of acrylic acid-based monomers, and it means a polymer that is not subjected to surface modification or surface crosslinking step as explained later.

**(Superabsorbent polymer composition)**

According to one embodiment of the present invention, provided is a superabsorbent polymer composition.

The superabsorbent polymer composition includes a superabsorbent polymer including a base polymer including a crosslinked polymer, in which an acrylic acid-based monomer having at least partially neutralized acidic groups and an internal crosslinking agent are crosslinking-polymerized, and a surface-crosslinked layer formed on the surface of the base polymer, in which the crosslinked polymer is additionally crosslinked via a surface crosslinking agent; and tannic acid; an iodine compound; and a chelating agent.

The acrylic acid-based monomer may be any monomer commonly used in the preparation of superabsorbent polymers. Specifically, the acrylic acid-based monomer may be a compound represented by the following Chemical Formula 1:

[Chemical Formula 1]   $R^1\text{-COOM}^1$

in Chemical Formula 1,

$R^1$ is a C2-C5 alkyl group containing an unsaturated bond, and
$M^1$ is a hydrogen atom, a monovalent or divalent metal, an ammonium group, or an organic amine salt.

Preferably, the acrylic acid-based monomer may include one or more selected from the group consisting of acrylic acid, methacrylic acid, and a monovalent metal salt thereof, a divalent metal salt thereof, an ammonium salt thereof, and an organic amine salt thereof.

The acrylic acid-based monomer has acidic groups, of which at least part may be neutralized. Preferably, those prepared by partially neutralizing the monomer with an alkaline substance, such as sodium hydroxide, potassium hydroxide, ammonium hydroxide, etc., may be used.

In this regard, the degree of neutralization of the monomer may be 40 mol% to 95 mol%, or 40 mol% to 80 mol%, or 45 mol% to 75 mol%. The range of the degree of neutralization may vary depending on the final physical properties. However, when the degree of neutralization is excessively high, the neutralized monomers are precipitated, and thus polymerization may not readily occur. On the contrary, when the degree of neutralization is excessively low, absorbency of the polymer greatly decreases, and furthermore, the polymer may exhibit hard-to-handle properties, like elastic rubber.

Meanwhile, in order to improve the physical properties of the polymer by polymerization of the acrylic acid-based monomer, it is performed in the presence of a crosslinking agent ("internal crosslinking agent"). The crosslinking agent is for internal crosslinking of the water-containing gel polymer, and may be used separately from a "surface crosslinking agent" described later.

As the internal crosslinking agent, any compound may be used as long as it enables introduction of crosslinking bonds during polymerization of the acrylic acid-based monomer. For non-limiting example, as the internal crosslinking agent, multifunctional crosslinking agents, such as N,N'-methylenebisacrylamide, trimethylolpropane tri(meth)acrylate, ethylene glycol di(meth)acrylate, polyethylene glycol di(meth)acrylate, propylene glycol di(meth)acrylate, polypropylene glycol di(meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentacrylate, glycerin tri(meth)acrylate, pentaerythritol tetraacrylate, triallylamine, allyl (meth)acrylate, ethylene glycol diglycidyl ether, propylene glycol, ethylene carbonate may be used alone or in combination of two or more thereof.

Such an internal crosslinking agent may be added at a concentration of 0.001% by weight to 1% by weight, or 0.01% by weight to 0.8% by weight, or 0.1% by weight to 0.7% by weight with respect to the monomer composition. In other words, when the concentration of the internal crosslinking agent is excessively low, the absorption rate of the polymer may decrease, and the gel strength may become weak, which is not preferred. On the contrary, when the concentration of the internal crosslinking agent is excessively high, the absorption capacity of the polymer may decrease, which is not preferred as an absorber.

In addition, the base polymer may further include additives such as thickeners, plasticizers, storage stabilizers, antioxidants, etc., as needed.

The surface-crosslinked layer is obtained by additionally crosslinking the crosslinked polymer via a surface crosslinking agent, and in this regard, the surface crosslinking agent is not particularly limited, as long as it is a surface crosslinking agent generally used for the surface crosslinking of superabsorbent polymers and is a compound capable of reacting with a functional group of the polymer.

Preferably, in order to improve the properties of superabsorbent polymer to be prepared, as the surface crosslinking agent, one or more selected from the group consisting of polyhydric alcohol; epoxy compounds; polyamine compounds; haloexpoy compounds; condensation products of haloexpoy compounds; oxazoline compounds; mono-, di- or polyoxazolidinone compounds; cyclic urea compounds; multivalent metal salts; and alkylene carbonate compounds may be used.

Specifically, as the examples of the polyhydric alcohol compounds, one or more selected from the group consisting of mono-, di-, tri-, tetra- or polyethylene glycol, monopropylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexanediol, and 1,2-cyclohexandimethanol may be used.

Further, as the epoxy compounds, ethylene glycol diglycidyl ether and glycidol, etc. may be used, and as the polyamine compounds, one or more selected from the group consisting of ethylenediamine, diethylenetriamine, triethylenetetraamine, tetraethylenepentamine, pentaethylenehexamine, polyethyleneimine and polyamide polyamine may be used.

Further, as the haloepoxy compounds, epichlorohydrin, epibromohydrin, and $\alpha$-methylepichlorohydrin may be used. Meanwhile, as the mono-, di- or polyoxazolidinone compounds, for example, 2-oxazolidinone may be used.

Further, as the alkylene carbonate compounds, ethylene carbonate, etc. may be used. These compounds may be used alone or in combinations. Meanwhile, in order to increase the efficiency of the surface crosslinking process, it is preferable to include one or more C2 to C10 polyhydric alcohol compounds among the surface crosslinking agents.

Specifically, the content of the surface crosslinking agent to be added may be appropriately selected according to the kind of surface crosslinking agents or reaction conditions, but commonly, it may be used in an amount of about 0.001 part by weight to about 5 parts by weight, preferably, about 0.01 part by weight to about 3 parts by weight, more preferably, about 0.05 parts by weight to about 2 parts by weight with respect to 100 parts by weight of the polymer.

When the content of the surface crosslinking agent is too small, a surface crosslinking reaction may hardly occur, and when the content exceeds 5 parts by weight with respect to 100 parts by weight of the polymer, deterioration of absorption capacity and physical properties may occur due to progression of excessive surface crosslinking reaction.

Meanwhile, the surface crosslinking agent may further include inorganic materials. As such inorganic materials, one or more inorganic materials selected from the group consisting of silica, clay, alumina, silica-alumina composite, titania, zinc oxide, and aluminum sulfate may be used. The inorganic material may be used in the form of powder or liquid, and particularly, in the form of alumina powder, silica-alumina powder, titania powder, or a nanosilica solution. Further, the inorganic material may be used in an amount of about 0.001 part by weight to about 1 part by weight with respect to 100 parts by weight of the base polymer.

The superabsorbent polymer composition of one embodiment of the present invention may include tannic acid, an iodine compound, and a chelating agent as deodorizing materials. The superabsorbent polymer composition including the tannic acid, iodine compound, and chelating agent is applied to hygiene products, it may have the effect of removing an unpleasant odor which is originally possessed by the liquid absorbed by the hygiene products and the effect of removing an unpleasant odor which may be additionally generated due to bacterial growth during use.

The tannic acid is a type of polyphenol found in fruit peels, vegetables, cacao, nuts, etc. The tannic acid has a hydroxyl group (-OH) in the molecule, and this hydroxyl group forms a hydrogen bond with the oxygen (O) or nitrogen (N) atom of a compound which generates an unpleasant odor, and therefore, the tannic acid may capture odor substances, thereby reducing the unpleasant odor.

The iodine compound may be an iodine solution ($I_2$) or a metal iodide salt. The iodine solution is in the form of $I_2$ dissolved in water, and the metal iodide salt may be added during the preparation process in the form in which one or more selected from the group consisting of CuI, NaI and KI and $I_2$ are dissolved in water together or in the form of a powder obtained by drying the aqueous solution. The iodine compound, like the tannic acid or chelating agent, may be added to the superabsorbent polymer to impart deodorizing properties. The iodine compound removes unpleasant odors by oxidizing unpleasant odor substances, and acts on most unpleasant odor substances, thereby showing general effects.

The superabsorbent polymer composition of one embodiment of the present invention may include a chelating agent. Hygiene products including the superabsorbent polymer composition may generate additional unpleasant odor due to the proliferation of bacteria by encounter between bacteria derived from the skin, etc., and absorbed liquid, and the chelating agent is able to inhibit the growth of these bacteria.

The chelating agent may include an amino acetate-based chelating agent. Specifically, the amino acetate-based chelating agent may include one or more selected from the group consisting of ethylenediaminetetraacetic acid (EDTA), L-glutamic acid diacetic acid (GLDA), methyl glycine diacetic acid (MGDA), hydroxyethylethylenediaminetriacetic acid (HEDTA), ethanol diglycine (EDG), diethylenetriaminepentaacetic acid (DTPA), and salts thereof. More specifically, the chelating agent may be ethylenediaminetetraacetic acid (EDTA) or L-glutamic acid diacetic acid (GLDA).

With respect to 100 parts by weight of the base polymer, the tannic acid may be included, based on the solid content, in an amount of more than 0.001 part by weight to 0.5 parts by weight, specifically, in an amount of more than 0.001 part by weight, 0.002 parts by weight or more, 0.003 parts by weight or more, 0.005 parts by weight or more, 0.01 part by weight or more, 0.015 parts by weight or more, or 0.02 parts by weight or more to 0.5 parts by weight or less, 0.3 parts by weight or less, 0.1 part by weight or less, 0.07 parts by weight or less, 0.05 parts by weight or less, 0.04 parts by weight or less, or 0.03 parts by weight or less.

Only when the tannic acid satisfies the above content range, it is possible to secure the maximum deodorizing ability with minimal deterioration in the physical properties of the superabsorbent polymer.

With respect to 100 parts by weight of the base polymer, the iodine compound may be included in an amount of 0.01 part by weight to 1.0 part by weight, specifically, in an amount of 0.01 part by weight or more, 0.03 parts by weight or more, 0.05 parts by weight or more, 0.07 parts by weight or more, or 0.1 part by weight or more to 1 part by weight or less, 0.8 parts by weight or less, 0.5 parts by weight or less, or 0.3 parts by weight or less.

Only when the iodine compound satisfies the above content range, it is possible to secure the maximum deodorizing ability with minimal deterioration in the physical properties of the superabsorbent polymer.

With respect to 100 parts by weight of the base polymer, the chelating agent may be included, based on the solid content, in an amount of 0.05 parts by weight to 4.0 parts by weight. Specifically, the chelating agent may be included, based on the solid content, in an amount of 0.05 parts by weight or more, 0.1 part by weight or more, 0.3 parts by weight or more, 0.5 parts by weight or more, 0.7 parts by weight or more, or 1.0 part by weight or more to 4.0 parts by weight or less, 3.0 parts by weight or less, 2.0 parts by weight or less, 1.5 parts by weight or less, or 1.3 parts by weight or less with respect to 100 parts by weight of the base polymer.

In order to achieve the deodorizing property through inhibiting the bacterial growth at the level desired to be achieved in the present invention and to minimize the deterioration of physical properties while maintaining the intrinsic absorption properties of the superabsorbent polymer, the chelating agent is preferably included in the above content range.

The superabsorbent polymer composition may further include an additional additive, in addition to the tannic acid, iodine compound, and chelating agent.

The additional additive may further include one or more additives selected from the group consisting of organic acids, glycerin, and zinc chloride.

The organic acid may be one or more selected from the group consisting of citric acid, glycine, acetic acid, formic acid, fumaric acid, lactic acid, and propionic acid. Specifically, the organic acid may be citric acid or glycine.

The organic acid may exhibit deodorizing effects in the superabsorbent polymer, like tannic acid. Further, the organic acid has the effect of neutralizing ammonia dissolved in urine. In particular, glycine may capture unpleasant odor substances by chemically reacting with the unpleasant odor substances. Unpleasant odor substances generally have a small molecular weight, and when they react with glycine, the unpleasant odor is reduced or eliminated, and thus the unpleasant odor may be effectively removed. In particular, glycine is effective in reducing unpleasant odors of aldehyde and ketone compounds.

Glycerin generally helps dissolve various deodorizing materials and exert deodorizing ability.

Zinc chloride acts as an antiseptic and preservative that inhibits bacterial metabolisms, and generally has antibacterial properties.

The tannic acid and iodine compound may be included separately from the superabsorbent polymer. As described in the preparation method below, tannic acid and iodine compound are mixed into the superabsorbent polymer after forming the surface-crosslinked layer on the surface of the base polymer. Therefore, the tannic acid and iodine compound may mainly exist outside the superabsorbent polymer particles.

The chelating agent may be included within the base polymer or separately from the superabsorbent polymer. As described in the preparation method below, the chelating agent is mixed in the coarse pulverization step of the step 2 or mixed with the superabsorbent polymer after forming the surface-crosslinked layer on the surface of the base polymer. In particular, when the chelating agent is mixed in the coarse pulverization step of the step 2, the chelating agent may be impregnated into and may exist in the base polymer.

**(Method of preparing superabsorbent polymer composition)**

According to one embodiment of the present invention, provided is a method of preparing the superabsorbent polymer composition.

The method of preparing the superabsorbent polymer composition includes the steps of:

forming a water-containing gel polymer by crosslinking polymerizing an acrylic acid-based monomer having at least partially neutralized acidic groups in the presence of an internal crosslinking agent and a polymerization initiator (step 1); preparing a base polymer including a crosslinked polymer by coarsely pulverizing, and then pulverizing and drying the water-containing gel polymer (step 2); preparing a mixture by mixing the base polymer with a surface crosslinking agent (step 3); and preparing a superabsorbent polymer, in which a surface-crosslinked layer is formed on the surface of the base polymer, by heat treatment of the mixture (step 4); wherein tannic acid and the iodine compound are mixed with the superabsorbent polymer of the step 4, in which the surface-crosslinked layer is formed, and the chelating agent is mixed in the coarse pulverization step of the step 2 or mixed with the superabsorbent polymer of the step 4, in which the surface-crosslinked layer is formed.

The method of preparing the superabsorbent polymer largely includes the step of preparing the water-containing gel polymer by polymerizing the acrylic acid-based monomer and the step of pulverizing the same. Further, in order to improve various physical properties of the superabsorbent polymer, a method of crosslinking the surface of the prepared superabsorbent polymer is used.

The present invention aims to provide a superabsorbent polymer composition with deodorizing ability by mixing tannic acid, the iodine compound, and the chelating agent with the surface-crosslinked superabsorbent polymer.

Hereinafter, each step of the present invention will be described in detail.

(Step 1)

The step 1 is a step of preparing the water-containing gel polymer, and specifically, a step of forming the water-containing gel polymer by crosslinking polymerizing a monomer composition including the acrylic acid-based monomer having at least partially neutralized acidic groups.

The acrylic acid-based monomer may be any monomer commonly used in the preparation of superabsorbent polymers. Specifically, the acrylic acid-based monomer may be a compound represented by the following Chemical Formula 1:

[Chemical Formula 1]         $R^1\text{-COOM}^1$

in Chemical Formula 1,

$R^1$ is a C2-C5 alkyl group containing an unsaturated bond, and
$M^1$ is a hydrogen atom, a monovalent or divalent metal, an ammonium group, or an organic amine salt.

Preferably, the acrylic acid-based monomer includes one or more selected from the group consisting of acrylic acid, methacrylic acid, and a monovalent metal salt thereof, a divalent metal salt thereof, an ammonium salt thereof, and an organic amine salt thereof.

The acrylic acid-based monomer has acidic groups, of which at least part may be neutralized. Preferably, those partially neutralized with an alkali material such as sodium hydroxide, potassium hydroxide, ammonium hydroxide, etc. may be used as the monomer.

In this regard, a degree of neutralization of the monomer may be 40 mol% to 95 mol%, or 40 mol% to 80 mol%, or 45 mol% to 75 mol%. The range of the degree of neutralization may vary depending on the final physical properties. However, when the degree of neutralization is excessively high, the neutralized monomers are precipitated, and thus polymerization may not readily occur. On the contrary, when the degree of neutralization is excessively low, absorbency of the polymer greatly decreases, and furthermore, the polymer may exhibit hard-to-handle properties, like elastic rubber.

The monomer composition may include a polymerization initiator which is commonly used in the preparation of superabsorbent polymers.

As the polymerization initiator, a thermal polymerization initiator or a photopolymerization initiator may be used depending on a polymerization method. However, even though the photopolymerization is performed, a certain amount of heat may be generated by UV irradiation or the like, and also generated with the polymerization reaction which is an exothermic reaction. Therefore, the thermal polymerization initiator may be further included.

Here, as the photopolymerization initiator, for example, one or more compounds selected from the group consisting of benzoin ether, dialkyl acetophenone, hydroxyl alkylketone, phenyl glyoxylate, benzyl dimethyl ketal, acyl phosphine, and α-aminoketone may be used. Among them, specific examples of acyl phosphine may include commercially available lucirin TPO, i.e., 2,4,6-trimethyl-benzoyl-trimethyl phosphine oxide. More various photopolymerization initiators are disclosed in "UV Coatings: Basics, Recent Developments and New Application (Elsevier, 2007)" written by Reinhold Schwalm, p115, which may be served as a reference.

As the thermal polymerization initiator, one or more compounds selected from the group consisting of persulfate-based initiators, azo-based initiators, hydrogen peroxide, and ascorbic acid may be used. Specifically, the persulfate-based initiators may be exemplified by sodium persulfate ($Na_2S_2O_8$), potassium persulfate ($K_2S_2O_8$), ammonium persulfate (($NH_4$)$_2S_2O_8$) or the like. Further, the azo-based initiators may be exemplified by 2,2-azobis(2-amidinopropane)dihydrochloride, 2,2-azobis-(N,N-dimethylene)isobutyramidine dihydrochloride, 2-(carbamoylazo)isobutyronitrile, 2,2-azobis[2-(2-imidazolin-2-yl)propane] dihydrochloride, 4,4-azobis-(4-cyanovaleric acid) or the like. More various thermal polymerization initiators are disclosed in "Principle of Polymerization (Wiley, 1981)" written by Odian, p203, which may be served as a reference.

Such a polymerization initiator may be added at a concentration of 0.001% by weight to 1% by weight, or 0.005% by weight to 0.1% by weight with respect to the monomer composition. In other words, when the concentration of the polymerization initiator is too low, the polymerization rate may become slow, and monomers remaining in the final product may be extracted in a large amount, which is not preferred. On the contrary, when the concentration of the polymerization initiator is too high, polymer chains constituting the network become short, and thus the content of water-soluble components is increased and physical properties of the polymer may deteriorate, such as a reduction in absorbency under pressure, which is not preferred.

Meanwhile, the polymerization of the monomer composition is performed in the presence of a crosslinking agent ("internal crosslinking agent") in order to improve physical properties of the polymer by the polymerization of the acrylic acid-based monomer. The crosslinking agent is for internal crosslinking of the water-containing gel polymer, and may be used separately from a "surface crosslinking agent" described later.

As the internal crosslinking agent, any compound may be used as long as it enables the introduction of crosslinking bonds during polymerization of the acrylic acid-based monomer. For non-limiting examples of the internal crosslinking agent, multifunctional crosslinking agents such as N,N'-methylenebisacrylamide, trimethylolpropane tri(meth)acrylate, ethylene glycol di(meth)acrylate, polyethylene glycol di(meth)acrylate, propylene glycol di(meth)acrylate, polypropylene glycol di(meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth) acrylate, tetraethylene glycol di(meth)acrylate, dipentaerythritol pentaacrylate, glycerin tri(meth)acrylate, pentaerythritol tetraacrylate, triallylamine, allyl (meth)acrylate, ethylene glycol diglycidyl ether, propylene glycol, or ethylene carbonate may be used alone or in combination of two or more thereof.

Such an internal crosslinking agent may be added at a concentration of 0.001% by weight to 1% by weight, or 0.01% by weight to 0.8% by weight, or 0.1% by weight to 0.7% by weight with respect to the monomer composition. In other words, when the concentration of the internal crosslinking agent is excessively low, the absorption rate of the polymer may decrease, and the gel strength may become weak, which is not preferred. On the contrary, when the concentration of the internal crosslinking agent is excessively high, the absorption capacity of the polymer may decrease, which is not preferred as an absorber.

In addition, the monomer composition may further include additives such as thickeners, plasticizers, storage stabilizers, antioxidants, etc., as needed.

Further, such a monomer composition may be prepared in a solution form, in which the raw materials, such as the above-described acrylic acid-based monomer, polymerization initiator, internal crosslinking agent, blowing agent, etc., are dissolved in a solvent.

In this regard, as the applicable solvent, any solvent may be used without limitations on the composition, as long as it is able to dissolve the above-described raw materials. For example, as the solvent, water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethylether, toluene, xylene, butyrolactone, carbitol, methyl cellosolve acetate, N,N-dimethylacetamide, or a mixture thereof may be used.

The formation of the water-containing gel polymer through the polymerization of the monomer composition may be performed by a common polymerization method, and the process is not particularly limited.

For non-limiting example, the polymerization method may be largely classified into thermal polymerization and photo-polymerization according to a polymerization energy source, and when the thermal polymerization is carried out, it may be carried out in a reactor like a kneader equipped with agitating spindles, and when the photo-polymerization is carried out, it may be carried out in a reactor equipped with a movable conveyor belt.

For example, the monomer composition is injected to the reactor like the kneader equipped with the agitating spindles, and thermal polymerization is carried out by providing hot air thereto or by heating the reactor, thereby obtaining the water-containing gel polymer. In this regard, the water-containing gel polymer discharged from an outlet of the reactor may be obtained as particles having a size of several centimeters or millimeters, according to the type of agitating spindles equipped in the reactor. Specifically, the water-containing gel polymer may be obtained in various forms according to a concentration of the monomer composition fed thereto, a feeding speed or the like, and the water-containing gel polymer having a (weight average) particle size of 2 mm to 50 mm may be generally obtained.

For another example, when the monomer composition is subjected to photopolymerization in the reactor equipped with the movable conveyor belt, the water-containing gel polymer may be obtained in a sheet shape. In this regard, the thickness of the sheet may vary according to the concentration of the monomer composition fed thereto and the feeding speed. The sheet is preferably controlled at a thickness of 0.5 cm to 10 cm in order to assure the production speed while allowing the entire sheet to be uniformly polymerized.

The water-containing gel polymer thus obtained by such a method may exhibit a water content of 40% by weight to 80% by weight. Here, the water content means a weight occupied by water with respect to the total weight of the water-containing gel polymer, which may be a value obtained by subtracting the weight of the dried polymer from the weight of the water-containing gel polymer. Specifically, the water content may be defined as a value calculated by measuring the weight loss due to evaporation of water in the polymer during the process of drying by raising the temperature of the polymer through infrared heating. At this time, the drying conditions may be set as follows: the temperature is increased from room temperature to 180°C and then the temperature is maintained at 180°C, and the total drying time is set to 20 minutes, including 5 minutes for the temperature rising step.

In the step 1, the water-containing gel polymer may be prepared in a sheet shape.

(Step 2)

The step 2 of the present invention is a step of forming a base polymer powder by coarsely pulverizing, drying, pulverizing, and classifying the sheet-shaped water-containing gel polymer prepared in the step 1.

Specifically, in order to increase the drying efficiency of the water-containing gel polymer as well as to affect various physical properties of the superabsorbent polymer, including the absorption rate, particularly, to improve the absorption rate of the superabsorbent polymer, by affecting the morphology of the superabsorbent polymer, the present invention may further include a step of coarsely pulverizing the water-containing gel polymer before drying the same. Hereinbelow, in order to distinguish it from pulverizing after drying, the term 'coarsely pulverizing' is used herein for convenience in relation to pulverizing before drying.

A pulverizer to be used for coarsely pulverizing may include, but there is no limitation in the configuration, specifically, any one selected from the group consisting of a vertical pulverizer, a turbo cutter, a turbo grinder, a rotary cutter mill, a cutter mill, a disc mill, a shred crusher, a crusher, a chopper, and a disc cutter, but is not limited to the above-described examples. Specifically, in the coarsely pulverizing of the present invention, a chopper may be used.

At this time, the step of coarsely pulverizing may be performed such that the water-containing gel polymer has a particle size of about 2 mm to about 20 mm. Pulverizing the water-containing gel polymer into a particle size of less than 2 mm is technically not easy due to a high water content of the water-containing gel polymer, and a phenomenon of agglomeration may occur between the pulverized particles. In contrast, when the water-containing gel polymer is pulverized into a particle size of larger than 20 mm, the effect of increasing the efficiency in the subsequent drying step may be poor. Specifically, the step of coarsely pulverizing may be performed using a chopper with holes of 15 mm to 17 mm.

Further, a chelating agent may be mixed with the water-containing gel polymer in the coarse pulverization step. Specifically, the chelating agent may be mixed in such a way that the sheet-shaped water-containing gel polymer prepared in the step 1 is finely cut, and chopping is performed while spraying the chelating agent.

The chelating agent may be mixed in an aqueous solution or a powder state.

The chelating agent may include an amino acetate-based chelating agent.

The amino acetate-based chelating agent may include one or more selected from the group consisting of ethylenediaminetetraacetic acid (EDTA), L-glutamic acid diacetic acid (GLDA), methyl glycine diacetic acid (MGDA), hydroxyethylethylenediaminetriacetic acid (HEDTA), ethanol diglycine (EDG), diethylenetriaminepentaacetic acid (DTPA), and salts thereof.

With respect to 100 parts by weight of the base polymer, the chelating agent may be included, based on the solid content, in an amount of 0.05 parts by weight to 4.0 parts by weight. Specifically, the chelating agent may be included, based on the solid content, in an amount of 0.05 parts by weight or more, 0.1 part by weight or more, 0.3 parts by weight or more, 0.5 parts by weight or more, 0.7 parts by weight or more, or 1.0 part by weight or more to 4.0 parts by weight or less, 3.0 parts by weight or less, 2.0 parts by weight or less, 1.5 parts by weight or less, or 1.3 parts by weight or less with respect to 100 parts by weight of the base polymer.

In order to inhibit the bacterial growth at the level desired to be achieved in the present invention and to minimize the deterioration of physical properties due to introduction of the chelating agent while maintaining the intrinsic absorption properties of the superabsorbent polymer, the chelating agent is preferably included in the above content range.

The drying may be performed at a temperature of 120°C to 250°C, 140°C to 200°C, or 150°C to 190°C. At this time, the drying temperature may be defined as the temperature of a heating medium supplied for drying or the internal temperature of a drying reactor containing the heating medium and the polymer in the drying process. When the drying temperature is low and the drying time is long, the process efficiency decreases. To prevent this, the drying temperature is preferably 120°C or higher. Further, when the drying temperature is higher than necessary, the surface of the water-containing gel polymer may be excessively dried, which may increase the generation of fine powder in the subsequent pulverizing step, and the physical properties of the final polymer may deteriorate. To prevent this, the drying temperature is preferably 250°C or lower.

At this time, the drying time in the drying step is not particularly limited, but may be adjusted to 20 minutes to 90 minutes under the drying temperature, considering the process efficiency and physical properties of the polymer.

The drying may be performed using a common medium, for example, may be performed through methods such as supplying hot air, infrared irradiation, microwave irradiation, or ultraviolet irradiation for the pulverized water-containing gel polymer, etc.

Further, the drying is preferably performed such that the dried polymer has a water content of 0.1% by weight to 10% by weight. In other words, when the water content of the dried polymer is less than 0.1% by weight, excessive drying may increase the production costs and may cause degradation of the crosslinked polymer, which is not preferred. Further, when the water content of the dried polymer is more than 10% by weight, defects may occur in subsequent processes, which is not preferred.

Subsequently, the dried water-containing gel polymer may be pulverized. This is a step to optimize the surface area of the base polymer powder and the superabsorbent polymer. The pulverization may be performed so that the particle size of the pulverized polymer is 150 $\mu$m to 850 $\mu$m.

In this regard, a pulverizer to be applicable may include common pulverizers such as a pin mill, a hammer mill, a screw mill, a roll mill, a disc mill, or a jog mill, etc.

Further, in order to manage the physical properties of the superabsorbent polymer to be finally commercialized, a step of selectively classifying particles having a particle size of 150 $\mu$m to 850 $\mu$m from the polymer particles, which are obtained through the pulverizing step, is performed.

Through the above classification step, a base polymer powder may be obtained. This base polymer powder may have a particle size of 150 $\mu$m to 850 $\mu$m, and may include 2% by weight or less or 1% by weight or less of fine powder having a particle size of less than 150 $\mu$m.

**(Step 3)**

The step 3 of the present invention is a step of mixing the base polymer powder prepared in the step 2 with a surface crosslinking agent.

The surface crosslinking agent used in the step 3 includes a surface crosslinking solution, and the surface crosslinking agent is not particularly limited, as long as it is a surface crosslinking agent generally used for surface crosslinking of superabsorbent polymers and is a compound capable of reacting with a functional group of the polymer.

Preferably, in order to improve the properties of superabsorbent polymer to be prepared, as the surface crosslinking agent, one or more selected from the group consisting of polyhydric alcohol; epoxy compounds; polyamine compounds; haloexpoy compounds; condensation products of haloexpoy compounds; oxazoline compounds; mono-, di- or polyoxazolidinone compounds; cyclic urea compounds; multivalent metal salts; and alkylene carbonate compounds may be used.

Specifically, as the examples of the polyhydric alcohol compounds, one or more selected from the group consisting of mono-, di-, tri-, tetra- or polyethylene glycol, monopropylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexanediol, and 1,2-cyclohexandimethanol may be used.

Further, as the epoxy compounds, ethylene glycol diglycidyl ether and glycidol, etc. may be used, and as the polyamine compounds, one or more selected from the group consisting of ethylenediamine, diethylenetriamine, triethylenetetraamine, tetraethylenepentamine, pentaethylenehexamine, polyethyleneimine and polyamide polyamine may be used.

Further, as the haloepoxy compounds, epichlorohydrin, epibromohydrin, and $\alpha$-methylepichlorohydrin may be used. Meanwhile, as the mono-, di- or polyoxazolidinone compounds, for example, 2-oxazolidinone, etc. may be used.

Further, as the alkylene carbonate compounds, ethylene carbonate, etc. may be used. These compounds may be used alone or in combinations. Meanwhile, in order to increase the efficiency of the surface crosslinking process, it is preferable to include one or more C2 to C10 polyhydric alcohol compounds among the surface crosslinking agents.

The content of the surface crosslinking agent to be added may be appropriately selected according to the kind of surface crosslinking agents to be added or reaction conditions. However, commonly, it may be used in an amount of about 0.001 part by weight to about 5 parts by weight, preferably, about 0.01 part by weight to about 3 parts by weight, more preferably, about 0.05 parts by weight to about 2 parts by weight with respect to 100 parts by weight of the polymer.

When the content of the surface crosslinking agent is too small, a surface crosslinking reaction may hardly occur, and when the content exceeds 5 parts by weight with respect to 100 parts by weight of the polymer, absorption capacity and physical properties may deteriorate, due to progression of excessive surface crosslinking reaction.

Meanwhile, the step of forming the surface-crosslinked layer may be performed by further including inorganic materials in the surface crosslinking agent. As such inorganic materials, one or more inorganic materials selected from the group consisting of silica, clay, alumina, silica-alumina composite, titania, zinc oxide, and aluminum sulfate may be used. The inorganic material may be used in the form of powder or liquid, and particularly, in the form of alumina powder, silica-alumina powder, titania powder, or a nanosilica solution. Further, the inorganic material may be used in an amount of about 0.001 part by weight to about 1 part by weight with respect to 100 parts by weight of the base polymer.

Meanwhile, with regard to a method of mixing the surface crosslinking agent with the base polymer, the method is not particularly limited as long as it is a method of evenly mixing the same with the base polymer, and an appropriate method is selected and used.

For example, a method of adding and mixing the surface crosslinking agent and the base polymer powder in a reactor, a method of spraying the surface crosslinking agent onto the base polymer, a method of mixing the base polymer and the surface crosslinking agent by continuously feeding them to a mixer which is continuously operated, etc. may be used.

In this regard, the surface crosslinking agent may be an aqueous solution, and when the solid content in the solution is 1% by weight or more, 3% by weight or more, 5% by weight or more, 10% by weight or more, or 50% by weight or less, 30% by weight or less, or 20% by weight or less, it is suitable for even distribution in the base polymer, and at the same time, it is possible to prevent the agglomeration of the base polymer.

**(Step 4)**

The step 4 is a step of further improving the physical properties of the superabsorbent polymer by reacting the base polymer with the surface crosslinking agent to form an interpenetrating polymer network on the surface of the crosslinked polymer included in the base polymer. Through this surface modification, a surface-crosslinked layer is formed on the surface of the pulverized base polymer particles.

The formation of the surface-crosslinked layer may be performed by a common method of increasing the crosslinking density of the surface of the polymer particles, for example, by a method of performing a crosslinking reaction by mixing a surface crosslinking agent including the surface crosslinking agent with the pulverized polymer and performing heat treatment.

The step 4 may be performed at a temperature of about 80°C to about 250°C. More specifically, the surface crosslinking process may be performed at a temperature of about 100°C to about 220°C, or about 110°C to about 200°C, or at a temperature of about 120°C to about 190°C for about 10 minutes to about 2 hours, or about 20 minutes to about 60 minutes. The crosslinking reaction temperature is lower than 160°C or the reaction time is too short, the surface crosslinking reaction does not occur properly and thus permeability may be lowered. When the temperature is higher than 200°C or the reaction time is too long, there may be a problem that the water retention capacity may be reduced.

A heating means for the surface crosslinking reaction is not particularly limited. Heating may be performed by providing a heating medium or by directly providing a heat source. In this regard, the kind of the applicable heating medium may be steam, hot air, a hot fluid such as hot oil or the like, but the present invention is not limited thereto. The temperature of the heating medium to be provided may be properly selected in consideration of the means of the heating medium, the heating rate, and the heating target temperature. Meanwhile, as the heat source to be directly provided, an electric heating or gas heating method may be used, but the present invention is not limited to the above-described examples.

Further, the superabsorbent polymer on which the surface-crosslinked layer is formed may be mixed with tannic acid, an iodine compound, and a chelating agent. The tannic acid and iodine compound chemically react with volatile unpleasant odor substances such as aldehydes, etc., thereby providing the superabsorbent polymer with the deodorizing ability.

With respect to 100 parts by weight of the base polymer, the tannic acid may be mixed, based on the solid content, in an amount of more than 0.001 part by weight to 0.5 parts by weight, specifically, in an amount of more than 0.001 part by weight, 0.002 parts by weight or more, 0.003 parts by weight or more, 0.005 parts by weight or more, 0.01 part by weight or more, 0.015 parts by weight or more, or 0.02 parts by weight or more to 0.5 parts by weight or less, 0.3 parts by weight or less, 0.1 part by weight or less, 0.07 parts by weight or less, 0.05 parts by weight or less, 0.04 parts by weight or less, or 0.03 parts by weight or less.

With respect to 100 parts by weight of the base polymer, the iodine compound may be mixed in an amount of 0.01 part by weight to 1.0 part by weight, specifically, in an amount of 0.01 part by weight or more, 0.03 parts by weight or more, 0.05 parts by weight or more, 0.07 parts by weight or more, or 0.1 part by weight or more to 1 part by weight or less, 0.8 parts by weight or less, 0.5 parts by weight or less, or 0.3 parts by weight or less.

In order to exhibit the deodorizing ability at the level desired to be achieved in the present invention while maintaining the intrinsic absorption properties of the superabsorbent polymer, tannic acid or iodine compound which is a deodorizing material is preferably included in the above content range.

The deodorizing material including the tannic acid and iodine compound may be mixed in an aqueous solution or a powder state with the base polymer, together with the surface crosslinking agent.

Further, descriptions of the type of the chelating agent to be mixed, the amount to be mixed, and the form to be mixed are as in the step 1.

Further, additional additives may be further mixed with the superabsorbent polymer on which the surface-crosslinked layer is formed. The additional additive may further include one or more additives selected from the group consisting of organic acids, glycerin, and zinc chloride.

The organic acid may be one or more selected from the group consisting of citric acid, glycine, acetic acid, formic acid, fumaric acid, lactic acid, and propionic acid. Specifically, the organic acid may be citric acid or glycine. The organic acid may exhibit deodorizing effects in the superabsorbent polymer, together with the deodorizing materials.

The organic acids, glycerin, and zinc chloride may be mixed in an aqueous solution in which water is used as a solvent, or in a powder state.

When the deodorizing materials and chelating agent are added as an aqueous solution to the superabsorbent polymer on which the surface-crosslinked layer is formed, an additional drying step may be further performed.

Hereinafter, preferred exemplary examples will be provided for better understanding of the present invention. However, the following exemplary examples are only for illustrating the present invention, but the present invention is not limited thereby.

The sources of reagents used in Comparative Examples and Examples described below are as follows.

Aqueous EDTA solution: Daejung Chemicals & Metals Co., Ltd.
Aqueous tannic acid solution: Samchun Chemical Co., Ltd.
Iodine solution: Bookwang Tech Co., Ltd.
Aqueous glycerin solution: Sigma-Aldrich
Aqueous glycine solution: Sigma-Aldrich

**Comparative Example 1**

100 g of acrylic acid, 0.37 g of N,N'-methylenebisacrylamide as a crosslinking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer solution composition having a monomer concentration of 45.8% by weight. The aqueous monomer solution composition was fed into a feed zone of a polymerization reactor which was equipped with a continuously moving conveyor belt, and then UV was irradiated (irradiation amount: 10 mW/cm$^2$) with a UV irradiation device while maintaining the polymerization atmosphere temperature at 80°C, and UV polymerization was performed for 2 minutes to prepare a sheet-shaped water-containing gel polymer.

The water-containing gel polymer was chopped using a meat chopper with holes of 16 mm. At this time, the chopped water-containing gel polymer had a water content of 47% by weight. Subsequently, the water-containing gel polymer was dried in a hot air dryer of 185°C for 30 minutes, and the dried water-containing gel polymer was pulverized with a pin mill, and subsequently, a polymer having a particles size of 150 μm to 850 μm was classified using a sieve, thereby preparing a base polymer.

Thereafter, a surface crosslinking agent (2.5 parts by weight of water, 0.1 part by weight of ethylene glycol diglycidyl ether (EX-810), 0.1 part by weight of aluminum sulfate 18 hydrate (Al-S), and 0.1 part by weight of silica (Aerosil A200)) was evenly mixed with 100 parts by weight of the prepared base polymer, and then a surface crosslinking reaction was performed at 140°C for 30 minutes. After completing the surface treatment, a superabsorbent polymer having a particle size of 150 μm to 850 μm was obtained using a sieve.

**Example 1**

100 g of acrylic acid, 0.37 g of N,N'-methylenebisacrylamide as a crosslinking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer solution composition having a monomer concentration of 45.8% by weight. The aqueous monomer solution composition was fed into a feed zone of a polymerization reactor which was equipped with a continuously moving conveyor belt, and then UV was irradiated (irradiation amount: 10 mW/cm$^2$) with a UV irradiation device while maintaining the polymerization atmosphere temperature at 80°C, and UV polymerization was performed for 2 minutes to prepare a sheet-shaped water-containing gel polymer.

The water-containing gel polymer was chopped using a meat chopper with holes of 16 mm. At this time, the chopped water-containing gel polymer had a water content of 47% by weight. Subsequently, the water-containing gel polymer was dried in a hot air dryer of 185°C for 30 minutes, and the dried water-containing gel polymer was pulverized with a pin mill, and subsequently, a polymer having a particles size of 150 μm to 850 μm was classified using a sieve, thereby preparing a base polymer.

Thereafter, a surface crosslinking agent (2.5 parts by weight of water, 0.1 part by weight of ethylene glycol diglycidyl ether (EX-810), 0.1 part by weight of aluminum sulfate 18 hydrate (Al-S), and 0.1 part by weight of silica (Aerosil A200)) was evenly mixed with 100 parts by weight of the prepared base polymer, and then a surface crosslinking reaction was performed at 140°C for 30 minutes. After completing the surface treatment, a superabsorbent polymer having a particle size of 150 μm to 850 μm was obtained using a sieve.

The surface-treated superabsorbent polymer was mixed with EDTA, tannic acid, an iodine solution, and glycerin in an aqueous solution state. The aqueous EDTA solution with a concentration of 40% was used. The aqueous tannic acid solution with a tannic acid solid content of 5 parts by weight, based on 100 parts by weight of the aqueous solution, was used. The iodine solution (liquid I$_2$) with a concentration of 0.1% was used.

EDTA with a solid content of 0.5 parts by weight with respect to 100 parts by weight of the base polymer, tannic acid with a solid content of 0.015 parts by weight with respect to 100 parts by weight of the base polymer, and the iodine solution of 0.1 part by weight with respect to 100 parts by weight of the base polymer were mixed. Then, the drying step was performed at 80°C for 25 minutes.

### Example 2

A surface-treated superabsorbent polymer was prepared in the same manner as in Example 1.

The surface-treated superabsorbent polymer was mixed with EDTA, tannic acid, and an iodine solution in an aqueous solution state. The aqueous EDTA solution with a solid content of 40% was used. The aqueous tannic acid solution with a tannic acid solid content of 5 parts by weight, based on 100 parts by weight of the aqueous solution, was used. The iodine solution (liquid $I_2$) with a concentration of 0.1% was used.

EDTA with a solid content of 0.5 parts by weight with respect to 100 parts by weight of the base polymer, tannic acid with a solid content of 0.05 parts by weight with respect to 100 parts by weight of the base polymer, and the iodine solution of 0.1 part by weight with respect to 100 parts by weight of the base polymer were mixed. Then, the drying step was performed at 80°C for 25 minutes.

### Example 3

A surface-treated superabsorbent polymer was prepared in the same manner as in Example 1.

The surface-treated superabsorbent polymer was mixed with EDTA, tannic acid, and an iodine solution in an aqueous solution state. The aqueous EDTA solution with a solid content of 40% was used. The aqueous tannic acid solution with a tannic acid solid content of 5 parts by weight, based on 100 parts by weight of the aqueous solution, was used. The iodine solution (liquid $I_2$) with a concentration of 0.1% was used.

EDTA with a solid content of 0.5 parts by weight with respect to 100 parts by weight of the base polymer, tannic acid with a solid content of 0.1 part by weight with respect to 100 parts by weight of the base polymer, and the iodine solution of 0.1 part by weight with respect to 100 parts by weight of the base polymer were mixed. Then, the drying step was performed at 80°C for 25 minutes.

### Example 4

A surface-treated superabsorbent polymer was prepared in the same manner as in Example 1.

The surface-treated superabsorbent polymer was mixed with EDTA, tannic acid, and an iodine solution in an aqueous solution state. The aqueous EDTA solution with a solid content of 40% was used. The aqueous tannic acid solution with a tannic acid solid content of 5 parts by weight, based on 100 parts by weight of the aqueous solution, was used. The iodine solution (liquid $I_2$) with a concentration of 0.1% was used.

EDTA with a solid content of 0.1 part by weight with respect to 100 parts by weight of the base polymer, tannic acid with a solid content of 0.015 parts by weight with respect to 100 parts by weight of the base polymer, and the iodine solution of 0.1 part by weight with respect to 100 parts by weight of the base polymer were mixed. Then, the drying step was performed at 80°C for 25 minutes.

### Example 5

A surface-treated superabsorbent polymer was prepared in the same manner as in Example 1.

The surface-treated superabsorbent polymer was mixed with EDTA, tannic acid, and an iodine solution in an aqueous solution state. The aqueous EDTA solution with a solid content of 40% was used. The aqueous tannic acid solution with a tannic acid solid content of 5 parts by weight, based on 100 parts by weight of the aqueous solution, was used. The iodine solution (liquid $I_2$) with a concentration of 0.1% was used.

EDTA with a solid content of 1 part by weight with respect to 100 parts by weight of the base polymer, tannic acid with a solid content of 0.015 parts by weight with respect to 100 parts by weight of the base polymer, and the iodine solution of 0.1 part by weight with respect to 100 parts by weight of the base polymer were mixed. Then, the drying step was performed at 80°C for 25 minutes.

### Example 6

A surface-treated superabsorbent polymer was prepared in the same manner as in Example 1.

The surface-treated superabsorbent polymer was mixed with EDTA, tannic acid, and an iodine solution in an aqueous solution state. The aqueous EDTA solution with a solid content of 40% was used. The aqueous tannic acid solution with a tannic acid solid content of 5 parts by weight, based on 100 parts by weight of the aqueous solution, was used. The iodine solution (liquid $I_2$) with a concentration of 0.1% was used.

EDTA with a solid content of 2 parts by weight with respect to 100 parts by weight of the base polymer, tannic acid with a solid content of 0.015 parts by weight with respect to 100 parts by weight of the base polymer, and the iodine solution of 0.1 part by weight with respect to 100 parts by weight of the base polymer were mixed. Then, the drying step was performed at 80°C for 25 minutes.

**Example 7**

A surface-treated superabsorbent polymer was prepared in the same manner as in Example 1.

The surface-treated superabsorbent polymer was mixed with EDTA, tannic acid, and an iodine solution in an aqueous solution state. The aqueous EDTA solution with a solid content of 40% was used. The aqueous tannic acid solution with a tannic acid solid content of 5 parts by weight, based on 100 parts by weight of the aqueous solution, was used. The iodine solution (liquid $I_2$) with a concentration of 0.1% was used.

EDTA with a solid content of 0.5 parts by weight with respect to 100 parts by weight of the base polymer, tannic acid with a solid content of 0.015 parts by weight with respect to 100 parts by weight of the base polymer, and the iodine solution of 0.05 parts by weight with respect to 100 parts by weight of the base polymer were mixed. Then, the drying step was performed at 80°C for 25 minutes.

**Example 8**

A surface-treated superabsorbent polymer was prepared in the same manner as in Example 1.

The surface-treated superabsorbent polymer was mixed with EDTA, tannic acid, and an iodine solution in an aqueous solution state. The aqueous EDTA solution with a solid content of 40% was used. The aqueous tannic acid solution with a tannic acid solid content of 5 parts by weight, based on 100 parts by weight of the aqueous solution, was used. The iodine solution (liquid $I_2$) with a concentration of 0.1% was used.

EDTA with a solid content of 0.5 parts by weight with respect to 100 parts by weight of the base polymer, tannic acid with a solid content of 0.015 parts by weight with respect to 100 parts by weight of the base polymer, and the iodine solution of 0.5 parts by weight with respect to 100 parts by weight of the base polymer were mixed. Then, the drying step was performed at 80°C for 25 minutes.

**Example 9**

A sheet-shaped water-containing gel polymer was prepared in the same manner as in Example 1.

The sheet-shaped water-containing gel polymer was finely cut, and the finely cut water-containing gel polymer was chopped (hole size of 16 mm) and mixed while spraying an aqueous EDTA solution (concentration of 40%) thereto. At this time, EDTA was mixed such that its solid content was 0.5 parts by weight, based on 100 parts by weight of the base polymer.

At this time, the chopped water-containing gel polymer had a water content of 47% by weight. Subsequently, the water-containing gel polymer was dried in a hot air dryer of 185°C for 30 minutes, and the dried water-containing gel polymer was pulverized with a pin mill, and subsequently, a polymer having a particles size of 150 μm to 850 μm was classified using a sieve, thereby preparing a base polymer.

Thereafter, a surface crosslinking agent (2.5 parts by weight of water, 0.1 part by weight of ethylene glycol diglycidyl ether (EX-810), 0.1 part by weight of aluminum sulfate 18 hydrate (Al-S), and 0.1 part by weight of silica (Aerosil A200)) was evenly mixed with 100 parts by weight of the prepared base polymer, and then a surface crosslinking reaction was performed at 140°C for 30 minutes. After completing the surface treatment, a superabsorbent polymer having a particle size of 150 μm to 850 μm was obtained using a sieve.

The surface-treated superabsorbent polymer was mixed with tannic acid and an iodine solution in an aqueous solution state. The aqueous tannic acid solution with a tannic acid solid content of 5 parts by weight, based on 100 parts by weight of the aqueous solution, was used. The iodine solution (liquid $I_2$) with a concentration of 0.1% was used.

Tannic acid with a solid content of 0.015 parts by weight with respect to 100 parts by weight of the base polymer, and the iodine solution of 0.5 parts by weight with respect to 100 parts by weight of the base polymer were mixed. Then, the drying step was performed at 80°C for 25 minutes.

**Example 10**

A surface-treated superabsorbent polymer was prepared in the same manner as in Example 1.

The surface-treated superabsorbent polymer was mixed with EDTA, tannic acid, and an iodine solution in an aqueous solution state. The aqueous EDTA solution with a solid content of 40% was used. The aqueous tannic acid solution with a tannic acid solid content of 5 parts by weight, based on 100 parts by weight of the aqueous solution, was used. The iodine solution (liquid $I_2$) with a concentration of 0.1% was used.

EDTA with a solid content of 0.5 parts by weight with respect to 100 parts by weight of the base polymer, tannic acid with a solid content of 0.015 parts by weight with respect to 100 parts by weight of the base polymer, and the iodine solution of 0.1 part by weight with respect to 100 parts by weight of the base polymer were mixed. Then, the drying step was performed at 80°C for 25 minutes.

**Example 11**

A surface-treated superabsorbent polymer was prepared in the same manner as in Example 1.

The surface-treated superabsorbent polymer was mixed with EDTA, tannic acid, an iodine solution, and glycine in an aqueous solution state. The aqueous EDTA solution with a solid content of 40% was used. The aqueous tannic acid solution with a tannic acid solid content of 5 parts by weight, based on 100 parts by weight of the aqueous solution, was used. The iodine solution (liquid $I_2$) with a concentration of 0.1% was used. The aqueous glycine solution with a concentration of 10% was used.

EDTA with a solid content of 0.5 parts by weight with respect to 100 parts by weight of the base polymer, tannic acid with a solid content of 0.015 parts by weight with respect to 100 parts by weight of the base polymer, the iodine solution of 0.1 part by weight with respect to 100 parts by weight of the base polymer, and glycine with a solid content of 0.018 parts by weight with respect to 100 parts by weight of the base polymer were mixed. Then, the drying step was performed at 80°C for 25 minutes.

**Example 12**

A surface-treated superabsorbent polymer was prepared in the same manner as in Example 1.

The surface-treated superabsorbent polymer was mixed with EDTA, tannic acid, an iodine solution, and glycerin in an aqueous solution state. The aqueous EDTA solution with a concentration of 40% was used. The aqueous tannic acid solution with a tannic acid solid content of 5 parts by weight, based on 100 parts by weight of the aqueous solution, was used. The iodine solution (liquid $I_2$) with a concentration of 0.1% was used. The aqueous glycerin solution with glycerin of 10 parts by weight, based on 100 parts by weight of the aqueous solution, was used.

EDTA with a solid content of 0.5 parts by weight with respect to 100 parts by weight of the base polymer, tannic acid with a solid content of 0.015 parts by weight with respect to 100 parts by weight of the base polymer, the iodine solution of 0.1 part by weight with respect to 100 parts by weight of the base polymer, and glycerin with a solid content of 0.03 parts by weight with respect to 100 parts by weight of the base polymer were mixed. Then, the drying step was performed at 80°C for 25 minutes.

**Comparative Example 2**

100 g of acrylic acid, 0.37 g of N,N'-methylenebisacrylamide as a crosslinking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer solution composition having a monomer concentration of 45.8% by weight. The aqueous monomer solution composition was fed into a feed zone of a polymerization reactor which was equipped with a continuously moving conveyor belt, and then UV was irradiated (irradiation amount: 10 mW/cm$^2$) with a UV irradiation device while maintaining the polymerization atmosphere temperature at 80°C, and UV polymerization was performed for 2 minutes to prepare a sheet-shaped water-containing gel polymer.

The sheet-shaped water-containing gel polymer was finely cut, and the finely cut water-containing gel polymer was chopped (hole size of 16 mm) and mixed while spraying an aqueous EDTA solution (concentration of 40%) thereto. At this time, EDTA was mixed such that its solid content was 0.5 parts by weight, based on 100 parts by weight of the base polymer.

At this time, the chopped water-containing gel polymer had a water content of 47% by weight. Subsequently, the water-containing gel polymer was dried in a hot air dryer of 185°C for 30 minutes, and the dried water-containing gel polymer was pulverized with a pin mill, and subsequently, a polymer having a particles size of 150 $\mu$m to 850 $\mu$m was classified using a sieve, thereby preparing a base polymer.

Thereafter, a surface crosslinking agent (2.5 parts by weight of water, 0.1 part by weight of ethylene glycol diglycidyl ether (EX-810), 0.1 part by weight of aluminum sulfate 18 hydrate (Al-S), and 0.1 part by weight of silica (Aerosil A200)) was evenly mixed with 100 parts by weight of the prepared base polymer, and then a surface crosslinking reaction was performed at 140°C for 30 minutes. After completing the surface treatment, a superabsorbent polymer having a particle size of 150 $\mu$m to 850 $\mu$m was obtained using a sieve.

## Comparative Example 3

A surface-treated superabsorbent polymer was prepared in the same manner as in Comparative Example 1.

The surface-treated superabsorbent polymer was mixed with glycerin in an aqueous solution state. The aqueous glycerin solution with glycerin of 10 parts by weight, based on 100 parts by weight of the aqueous solution, was used.

0.03 parts by weight of glycerin with respect to 100 parts by weight of the base polymer was mixed. Then, the drying step was performed at 80°C for 25 minutes.

## Comparative Example 4

A surface-treated superabsorbent polymer was prepared in the same manner as in Comparative Example 1.

The surface-treated superabsorbent polymer was mixed with tannic acid and glycerin in an aqueous solution state. The aqueous tannic acid solution with a tannic acid solid content of 5 parts by weight, based on 100 parts by weight of the aqueous solution, was used. The aqueous glycerin solution with glycerin of 10 parts by weight, based on 100 parts by weight of the aqueous solution, was used.

Tannic acid with a solid content of 0.015 parts by weight with respect to 100 parts by weight of the base polymer, and 0.03 parts by weight of glycerin with respect to 100 parts by weight of the base polymer were mixed. Then, the drying step was performed at 80°C for 25 minutes.

## Comparative Example 5

A surface-treated superabsorbent polymer was prepared in the same manner as in Comparative Example 1.

The surface-treated superabsorbent polymer was mixed with EDTA, and tannic acid in an aqueous solution state. The aqueous EDTA solution with a concentration of 40% was used. The aqueous tannic acid solution with a tannic acid solid content of 5 parts by weight, based on 100 parts by weight of the aqueous solution, was used.

EDTA with a solid content of 0.5 parts by weight with respect to 100 parts by weight of the base polymer, and tannic acid with a solid content of 0.015 parts by weight with respect to 100 parts by weight of the base polymer were mixed. Then, the drying step was performed at 80°C for 25 minutes.

## Comparative Example 6

A surface-treated superabsorbent polymer was prepared in the same manner as in Comparative Example 1.

The surface-treated superabsorbent polymer was mixed with glycine in an aqueous solution state. The aqueous glycine solution with a concentration of 10% was used.

0.018 parts by weight of glycine with respect to 100 parts by weight of the base polymer was mixed. Then, the drying step was performed at 80°C for 25 minutes.

## Comparative Example 7

A surface-treated superabsorbent polymer was prepared in the same manner as in Comparative Example 1.

The surface-treated superabsorbent polymer was mixed with EDTA and glycerin in an aqueous solution state. The aqueous EDTA solution with a concentration of 40% was used. The aqueous glycerin solution with a glycerin content of 10 parts by weight, based on 100 parts by weight of the aqueous solution, was used.

EDTA with a solid content of 0.5 parts by weight with respect to 100 parts by weight of the base polymer, and glycerin of 0.03 parts by weight with respect to 100 parts by weight of the base polymer were mixed. Then, the drying step was performed at 80°C for 25 minutes.

## Comparative Example 8

A surface-treated superabsorbent polymer was prepared in the same manner as in Comparative Example 1.

The surface-treated superabsorbent polymer was mixed with tannic acid in an aqueous solution state. The aqueous tannic acid solution with a tannic acid solid content of 5 parts by weight, based on 100 parts by weight of the aqueous solution, was used.

Tannic acid with a solid content of 0.015 parts by weight with respect to 100 parts by weight of the base polymer was mixed. Then, the drying step was performed at 80°C for 25 minutes.

**Comparative Example 9**

A surface-treated superabsorbent polymer was prepared in the same manner as in Comparative Example 1.
The surface-treated superabsorbent polymer was mixed with an iodine solution (liquid $I_2$). An aqueous solution with a concentration of 0.1% was used as the iodine solution.
The iodine solution of 0.1 part by weight with respect to 100 parts by weight of the base polymer was mixed. Then, the drying step was performed at 80°C for 25 minutes.

**Comparative Example 10**

A surface-treated superabsorbent polymer was prepared in the same manner as in Comparative Example 1.
The surface-treated superabsorbent polymer was mixed with tannic acid and an iodine solution (liquid $I_2$). The aqueous tannic acid solution with a tannic acid solid content of 5 parts by weight, based on 100 parts by weight of the aqueous solution, was used. An aqueous solution with a concentration of 0.1% was used as the iodine solution.
Tannic acid with a solid content of 0.015 parts by weight with respect to 100 parts by weight of the base polymer was mixed. The iodine solution of 0.1 part by weight with respect to 100 parts by weight of the base polymer was mixed. Then, the drying step was performed at 80°C for 25 minutes.

**Comparative Example 11**

A surface-treated superabsorbent polymer was prepared in the same manner as in Comparative Example 1.
The surface-treated superabsorbent polymer was mixed with an aqueous EDTA solution and an iodine solution (liquid $I_2$). An aqueous solution with a concentration of 40% was used as the aqueous EDTA solution. An aqueous solution with a concentration of 0.1% was used as the iodine solution.
EDTA with a solid content of 0.5 parts by weight with respect to 100 parts by weight of the base polymer was mixed. The iodine solution of 0.1 part by weight with respect to 100 parts by weight of the base polymer was mixed. Then, the drying step was performed at 80°C for 25 minutes.
The conditions of Examples and Comparative Examples are summarized in Tables 1 and 2 below.

[Table 1]

| Section | Chelating agent | | | Tannic acid | | | Iodine com pound | | | Additional additive | | |
|---|---|---|---|---|---|---|---|---|---|---|---|---|
| | Type | Mixing amount | Mixing time | tannic acid | Mixing amount | Mixing time | Type | Mixing amount | Mixing time | Type | Mixing amount | Mixing time |
| Example 1 | ED TA | 0.5 | after surface crosslinking | tannic acid | 0.01 5 | after surface crosslinking | iodine solution | 0.1 | after surface crosslinking | | | |
| Example 2 | ED TA | 0.5 | after surface crosslinking | tannic acid | 0.05 | after surface crosslinking | iodine solution | 0.1 | after surface crosslinking | | | |
| Exam ple 3 | ED TA | 0.5 | after surface crosslinkin g | tann ic acid | 0.1 | after surface crosslink ing | iodin e soluti on | 0.1 | after surface crosslink ing | | | |
| Exam ple 4 | ED TA | 0.1 | after surface crosslinkin g | tann ic acid | 0.01 5 | after surface crosslink ing | iodin e soluti on | 0.1 | after surface crosslink ing | | | |
| Exam ple 5 | ED TA | I | after surface crosslinkin g | tann ic acid | 0.01 5 | after surface crosslink ing | iodin e soluti on | 0.1 | after surface crosslink ing | | | |
| Exam ple 6 | ED TA | 2 | after surface crosslinkin g | tann ic acid | 0.01 5 | after surface crosslink ing | iodin e soluti on | 0.1 | after surface crosslink ing | | | |
| Exam ple 7 | ED TA | 0.5 | after surface crosslinkin g | tann ic acid | 0.01 5 | after surface crosslink ing | iodin e soluti on | 0.05 | after surface crosslink ing | | | |
| Exam ple 8 | ED TA | 0.5 | after surface crosslinkin g | tann ic acid | 0.01 5 | after surface crosslink ing | iodin e soluti on | 0.5 | after surface crosslink ing | | | |
| Exam ple 9 | ED TA | 0.5 | after polymeriza tion | tann ic acid | 0.01 5 | after surface crosslink ing | iodin e soluti on | 0.5 | after surface crosslink ing | | | |
| Exam ple 10 | ED TA | 0.5 | after surface crosslinkin g | tann ic acid | 0.01 5 | after surface crosslink ing | iodin e soluti on | 0.1 | after surface crosslink ing | | | |
| Exam ple 11 | ED TA | 0.5 | after surface crosslinkin g | tann ic acid | 0.01 5 | after surface crosslink ing | iodin e soluti on | 0.1 | after surface crosslink ing | glyci ne | 0.01 8 | after sur- face cross- link ing |
| Exam ple 12 | ED TA | 0.5 | after surface crosslinkin g | tann ic acid | 0.01 5 | after surface crosslink ing | iodin e soluti on | 0.1 | after surface crosslink ing | glyce rin | 0.03 | after sur- face cross- link ing |

18

EP 4 660 233 A1

[Table 2]

| Section | Chelating agent | | | Tannic acid | | | Iodine compound | | | Additional additive | | |
|---|---|---|---|---|---|---|---|---|---|---|---|---|
| | Type | Mixing amount | Mixing time | Tannic acid | Mixing amount | Mixing time | Type | Mixing amount | Mixing time | Type | Mixing amount | Mixing time |
| Comparative Example 1 | - | - | - | - | - | - | - | - | - | - | - | - |
| Comparative Example 2 | EDTA | 0.5 | after surface crosslinking | - | - | - | - | - | - | - | - | - |
| Comparative Example 3 | - | - | - | - | - | - | - | | | glycerin | 0.03 | after surface crosslinking |
| Comparative Example 4 | - | - | - | tannic acid | 0.015 | after surface crosslinking | - | - | - | glycerin | 0.03 | after surface crosslinking |
| Comparative Example 5 | EDTA | 0.5 | after surface crosslinking | tannic acid | 0.015 | after surface crosslinking | - | - | - | - | - | - |
| Comparative Example 6 | - | - | - | - | - | - | - | - | - | glycine | 0.018 | after surface crosslinking |
| Comparative Example 7 | EDTA | 0.5 | after surface crosslinking | - | - | - | - | - | - | glycerin | 0.03 | after surface crosslinking |
| Comparative Example 8 | - | - | - | tannic acid | 0.015 | after surface crosslinking | - | - | - | - | - | - |
| Comparative Example 9 | - | - | - | - | - | - | iodine solution | 0.1 | after surface crosslinking | - | - | - |
| Comparative Example 10 | - | - | - | tannic acid | 0.015 | after surface crosslinking | iodine solution | 0.1 | after surface crosslinking | - | - | - |
| Comparative Example 11 | EDTA | 0.5 | after surface crosslinking | - | - | - | iodine solution | 0.1 | after surface crosslinking | - | - | - |

**Experimental Example**

For the superabsorbent polymer compositions prepared in Examples and Comparative Examples, each physical property was measured by the following method.

1) **Bacterial inhibition rate**

50 ml of artificial urine, in which 3000 CFU/ml of *E. Coli* was inoculated, was put in 2 g of the EDTA-free superabsorbent polymer of Comparative Example 1, and incubated in an incubator at 35°C for 12 hours. The sample, of which incubation was completed, was washed well with 150 ml of saline solution (0.9 wt% aqueous sodium chloride solution), cultured on a nutrient broth agar (BD DIFCO.) plate, and Colony Forming Unit (CFU/ml) was measured, which was determined as the physical property of the control group.

2 g of each of the superabsorbent polymers prepared in Examples or Comparative Examples was added to 50 ml of artificial urine, in which 3000 CFU/ml of *E. Coli* was inoculated, and incubated in an incubator at 35°C for 12 hours. To each sample, of which incubation was completed, 150 ml of saline solution (0.9 wt% aqueous sodium chloride solution) was added and evenly mixed with shaking for 1 minute. The resulting dilution was spread on a nutrient broth agar (BD DIFCO.) plate, and incubated in an incubator at 30°C for 24 hours, and then Colony Forming Unit (CFU/ml) was measured.

Each of the measurement results was used to calculate a bacterial inhibition rate defined by the following Equation 1, and based on this, antibacterial activities of the superabsorbent polymers of Examples and Comparative Examples were evaluated:

[Equation 1]

$$\text{Bacterial growth inhibition rate} = [1 - \{CFU(12hr) / CFU\ control(12hr)\}] * 100\ (\%)$$

in Equation 1,

CFU(12hr) represents the number of individuals of proliferated bacteria per unit volume of artificial urine (CFU/ml), which was obtained by adding the superabsorbent polymer to the artificial urine inoculated with bacteria of *E. Coli,* and then incubating for 12 hours at 35°C, and

CFU control(12hr) represents the number of individuals of proliferated bacteria per unit volume of artificial urine (CFU/ml), which was obtained by incubating artificial urine inoculated with the bacteria in the EDTA-free superabsorbent polymer under the same conditions, i.e., the number of individuals of bacteria of the control group per unit volume of artificial urine (CFU/ml).

2) **Deodorization rate**

The deodorization rate was measured using a sorbent tube test. 3-methylbutanal as an aldehyde-based unpleasant odor substance, and dimethyltrisulfide (DMTS) as a sulfur compound-based unpleasant odor substance were selected and their deodorizing ability was tested.

- Sorbent tube test: 1 g of the superabsorbent polymer was put in a 500 mL glass bottle, and then 25 mL of the unpleasant odor substance was injected. Thereafter, aging was performed for 3 hours in a constant temperature chamber and collection was performed for 20 minutes. At this time, the temperature of the constant temperature chamber was 35°C and the $N_2$ flow rate was 250 mL/min. The unpleasant odor pushed out was then adsorbed to the connected sorbent tube, which was collected twice for the same sample. The collection results were analyzed by GC to confirm the results.
- Deodorizing ability (%) = (Amount of unpleasant odor of reference sample (superabsorbent polymer of Comparative Example 1) measured by GC - Amount of unpleasant odor of sample measured by GC) / Amount of unpleasant odor of reference sample (superabsorbent polymer of Comparative Example 1) measured by GC X 100(%)

3) **Centrifuge Retention Capacity (CRC)**

The water retention capacity by absorbency under no load was measured for each polymer in accordance with European Disposables and Nonwovens Association (EDANA) standard EDANA WSP 241.3.

In detail, polymers in a particle size range of 300 $\mu$m to 600 $\mu$m were classified from each of the polymers obtained through Examples and Comparative Examples. The polymer $W_0$(g) (about 0.2 g) was evenly put in a nonwoven fabric-made bag, followed by sealing. Then, the bag was immersed in a physiological saline solution (0.9% by weight) at room temperature. After 30 minutes, water was removed from the bag using a centrifuge at 250 G for 3 minutes, and the weight $W_2$(g) of the bag was then measured. Further, the same procedure was carried out without using the polymer, and then the resultant weight $W_1$(g) was measured.

By using the respective weights thus obtained, CRC (g/g) was calculated according to the following Equation 1.

[Equation 1]

$$CRC\ (g/g) = \{[W_2(g) - W_1(g)]/W_0(g)\} - 1$$

### 4) Absorbency under Pressure (AUP)

Absorbency under pressure of 0.7 psi of the superabsorbent polymers of Examples and Comparative Examples was measured in accordance with the EDANA method WSP 242.3.

First, when measuring absorbency under pressure, the classified polymer used at the time of CRC measurement was used.

In detail, a 400 mesh steel net was installed in the bottom of a plastic cylinder having an internal diameter of 25 mm. The superabsorbent polymer $W_0$(g) was uniformly scattered on the steel net under conditions of room temperature and humidity of 50%. A piston capable of uniformly providing a load of 0.7 psi was placed thereon, in which an external diameter of the piston was slightly smaller than 25 mm, there was no gab between the internal wall of the cylinder and the piston, and the jig-jog of the cylinder was not interrupted. At this time, the weight $W_3$(g) of the apparatus was measured.

After placing a glass filter having a diameter of 90 mm and a thickness of 5 mm in a petri dish having a diameter of 150 mm, a physiological saline solution consisting of 0.9% by weight of sodium chloride was poured until the surface level of the physiological saline solution became equal to the upper surface of the glass filter. A sheet of filter paper having a diameter of 90 mm was placed on the glass filter. The measurement apparatus was mounted on the filter paper, thereby getting the liquid absorbed under the load for 1 hour. 1 hour later, after lifting the measurement apparatus up, the weight $W_4$ (g) was measured. Absorbency under pressure (g/g) was calculated using the obtained weights according to the following Equation 2.

[Equation 2]

$$AUP(g/g) = [W_4(g) - W_3(g)]/W_0(g)$$

### 5) Gel Strength (N)

From the superabsorbent polymers, those having a particle size of 150 $\mu$m to 850 $\mu$m were taken, 2.5 g of the superabsorbent polymer was immersed in 50 g of ascorbic acid brine, and allowed to swell in an oven at 40 °C for 2 hours and 4 hours, then gel strength of the swollen superabsorbent polymer was measured using a tensile compression tester.

In detail, the swollen superabsorbent polymer was measured using a digital force gauge FGP-2, which is a tensile compression tester, and the peak value of the force (N) applied to the tip as the tip was penetrated was measured three times according to the following conditions. Then, the arithmetic mean value was taken as the gel strength (unit: N).

Tip size: terminal diameter of 10±0.1 mm

Beaker size: 50±0.1 mm

Penetration speed: 500±0.5 mm/min

The results of the experiment are shown in Table 3.

[Table 3]

| | Bacterial inhibition rate (E.coli, %) | Deodorization rate (%) | | Absorption performances | | Gel strength | |
|---|---|---|---|---|---|---|---|
| | | Aldehyde series | Sulfur compound series | CRC | 0.7 AUP | Swollen for 2 hr | Swollen for 4 hr |
| Example 1 | 99 | 80 | 82 | 36.9 | 17.2 | 1.06 | 0.82 |
| Example 2 | 99 | 86 | 88 | 36.4 | 16.7 | - | - |
| Example 3 | 99 | 92 | 90 | 36 | 16.1 | - | - |
| Example 4 | 82 | 76 | 80 | 37.2 | 17.4 | - | - |
| Example 5 | 99.9 | 81 | 85 | 36.1 | 16 | - | - |
| Example 6 | 99.9 | 81 | 86 | 35.5 | 15.5 | - | - |
| Example 7 | 99 | 70 | 73 | 37 | 17.2 | - | - |
| Example 8 | 99 | 85 | 87 | 36.5 | 16.6 | - | - |
| Example 9 | 99 | 81 | 81 | 37 | 17.3 | - | - |
| Example 10 | 76 | 75 | 78 | 36.5 | 16.9 | - | - |
| Example 11 | 98 | 85 | 86 | 36.4 | 16.8 | - | - |
| Example 12 | 98 | 82 | 84 | 36.3 | 16.9 | - | - |
| Comparative Example 1 | 0 | 0 | 0 | 37.5 | 17.9 | 0.72 | 0.63 |
| Comparative Example 2 | 98 | 0 | 10 | 37.0 | 16.8 | 0.81 | 0.68 |
| Comparative Example 3 | 0 | 30 | 25 | 37.0 | 17.0 | - | - |
| Comparative Example 4 | 0 | 61 | 59 | 37.1 | 17.3 | - | - |
| Comparative Example 5 | 96 | 63 | 65 | 37.0 | 16.5 | - | - |
| Comparative Example 6 | 0 | 15 | 12 | 36.5 | 17.5 | - | - |
| Comparative Example 7 | 97 | 15 | 15 | 37.0 | 16.9 | - | - |
| Comparative Example 8 | 0 | 55 | 54 | 37.3 | 17.5 | 0.96 | 0.62 |
| Comparative Example 9 | 45 | 15 | 25 | 37.5 | 17.4 | - | - |
| Comparative Example 10 | 48 | 67 | 68 | 37.2 | 17.4 | - | - |
| Comparative Example 11 | 99 | 15 | 25 | 37.1 | 17 | - | - |

According to the results in Table 3, it was confirmed that Examples had the excellent bacterial inhibition rate and deodorization rate while maintaining absorbency at a similar level, as compared to Comparative Example 1 without additives. It was also confirmed that Example 1 had the excellent gel strength, as compared to Comparative Examples 1, 2, and 8.

Further, Comparative Example 2, in which only the chelating agent was used without the deodorizing materials, showed the bacterial inhibition rate at a similar level while showing the very poor deodorization rate, and Comparative Examples, in which only the deodorizing materials were used without the chelating agent, did not inhibit bacteria at all, and their deodorization rate also tended to be low, as compared to those of Examples.

In contrast, it was confirmed that the superabsorbent polymers of Examples, unlike those of Comparative Examples, showed no deterioration in absorbency which is an intrinsic physical property of the superabsorbent polymer while having the excellent bacterial inhibition rate and deodorization rate, and maintained the excellent gel strength.

It was assumed that iodine ions of the iodine compound included in Examples change the ascorbic acid component in the urine and body fluids and blocks the gelinhibiting function of ascorbic acid. In addition, since the chelating agent is known to contribute in some degree to the gel strength improvement effect by combining with metal ions, it was confirmed that the gel strength was improved by its combination.

**Claims**

1. A superabsorbent polymer composition comprising:

   a superabsorbent polymer including a base polymer including a crosslinked polymer, wherein an acrylic acid-based monomer having at least partially neutralized acidic groups and an internal crosslinking agent are crosslinking-polymerized, and a surface-crosslinked layer formed on the surface of the base polymer, wherein the crosslinked polymer is additionally crosslinked via a surface crosslinking agent;
   tannic acid;
   an iodine compound; and
   a chelating agent.

2. The superabsorbent polymer composition of claim 1, wherein the tannic acid is included, based on the solid content, in an amount of more than 0.001 part by weight to 0.5 parts by weight with respect to 100 parts by weight of the base polymer.

3. The superabsorbent polymer composition of claim 1, wherein the iodine compound is included in an amount of 0.01 part by weight to 1.0 part by weight with respect to 100 parts by weight of the base polymer.

4. The superabsorbent polymer composition of claim 1, wherein the chelating agent is included, based on the solid content, in an amount of 0.05 parts by weight to 4.0 parts by weight with respect to 100 parts by weight of the base polymer.

5. The superabsorbent polymer composition of claim 1, wherein the chelating agent includes an amino acetate-based chelating agent.

6. The superabsorbent polymer composition of claim 5, wherein the amino acetate-based chelating agent includes one or more selected from the group consisting of ethylenediaminetetraacetic acid (EDTA), L-glutamic acid diacetic acid (GLDA), methyl glycine diacetic acid (MGDA), hydroxyethylethylenediaminetriacetic acid (HEDTA), ethanol diglycine (EDG), diethylenetriaminepentaacetic acid (DTPA), and salts thereof.

7. The superabsorbent polymer composition of claim 1, further comprising one or more additives selected from the group consisting of organic acids, glycerin, and zinc chloride.

8. The superabsorbent polymer composition of claim 1, wherein the tannic acid and the iodine compound are included separately from the superabsorbent polymer, and
   the chelating agent is included within the base polymer or separately from the superabsorbent polymer.

9. A method of preparing a superabsorbent polymer composition, the method comprising the steps of:

   forming a water-containing gel polymer by crosslinking polymerizing an acrylic acid-based monomer having at least partially neutralized acidic groups in the presence of an internal crosslinking agent and a polymerization initiator (step 1);
   preparing a base polymer including a crosslinked polymer by coarsely pulverizing, drying, and pulverizing the water-containing gel polymer (step 2);
   preparing a mixture by mixing the base polymer with a surface crosslinking agent (step 3); and
   preparing a superabsorbent polymer, in which a surface-crosslinked layer is formed on the surface of the base polymer, by heat treatment of the mixture (step 4);
   wherein tannic acid and an iodine compound are mixed with the superabsorbent polymer of the step 4, on which the surface-crosslinked layer is formed, and a chelating agent is mixed in the coarse pulverization step of the step 2 or mixed with the superabsorbent polymer of the step 4, on which the surface-crosslinked layer is formed.

10. The method of claim 9, wherein the tannic acid is mixed, based on the solid content, in an amount of more than 0.001 part by weight to 0.1 part by weight with respect to 100 parts by weight of the base polymer.

11. The method of claim 9, wherein the iodine compound is mixed in an amount of 0.01 part by weight to 1.0 part by weight with respect to 100 parts by weight of the base polymer.

12. The method of claim 9, wherein the chelating agent is mixed, based on the solid content, in an amount of 0.05 parts by weight to 4.0 parts by weight with respect to 100 parts by weight of the base polymer.

13. The method of claim 9, wherein the chelating agent includes an amino acetate-based chelating agent.

14. The method of claim 13, wherein the amino acetate-based chelating agent includes one or more selected from the group consisting of ethylenediaminetetraacetic acid (EDTA), L-glutamic acid diacetic acid (GLDA), methyl glycine diacetic acid (MGDA), hydroxyethylethylenediaminetriacetic acid (HEDTA), ethanol diglycine (EDG), diethylenetriaminepentaacetic acid (DTPA), and salts thereof.

15. The method of claim 9, wherein after the step 4, the surface-crosslinked layerformed superabsorbent polymer is further mixed with one or more additives selected from the group consisting of organic acids, glycerin, and zinc chloride.

## INTERNATIONAL SEARCH REPORT

International application No.

**PCT/KR2024/007762**

| A. | CLASSIFICATION OF SUBJECT MATTER |
|---|---|

**C08K 5/1545**(2006.01)i; **C08K 5/17**(2006.01)i; **C08K 5/09**(2006.01)i; **C08K 5/053**(2006.01)i; **C08K 3/16**(2006.01)i; **C08J 3/24**(2006.01)i

According to International Patent Classification (IPC) or to both national classification and IPC

| B. | FIELDS SEARCHED |
|---|---|

Minimum documentation searched (classification system followed by classification symbols)

C08K 5/1545(2006.01); A61L 15/00(2006.01); A61L 15/42(2006.01); A61L 15/46(2006.01); A61L 9/01(2006.01); A61L 9/012(2006.01); C08F 2/10(2006.01); C08J 3/075(2006.01); C08J 3/12(2006.01); C08J 3/24(2006.01); C08J 7/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 고흡수성 수지(superabsorbent resin), 탄닌산(tannic acid), 요오드 화합물(iodine compound), 킬레이트제(chelating agent), 악취 제거(odor removal)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2010-0074230 A (EVONIK STOCKHAUSEN GMBH) 01 July 2010 (2010-07-01)<br>See paragraphs [0038], [0040], [0065], [0076]-[0080], [0106] and [0143]-[0148]; and claim 1. | 1-15 |
| A | EP 1358894 A1 (SCA HYGIENE PRODUCTS AB) 05 November 2003 (2003-11-05)<br>See entire document. | 1-15 |
| A | KR 10-2021-0058928 A (NIPPON SHOKUBAI CO., LTD.) 24 May 2021 (2021-05-24)<br>See entire document. | 1-15 |
| A | KR 10-2022-0082510 A (LG CHEM, LTD.) 17 June 2022 (2022-06-17)<br>See entire document. | 1-15 |
| A | US 2010-0035757 A1 (FURNO, Franck et al.) 11 February 2010 (2010-02-11)<br>See entire document. | 1-15 |

☐ Further documents are listed in the continuation of Box C. ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 September 2024** | **11 September 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2024/007762**

| Patent document cited in search report | | | Publication date (day/month/year) | Patent family member(s) | | | Publication date (day/month/year) |
|---|---|---|---|---|---|---|---|
| KR | 10-2010-0074230 | A | 01 July 2010 | CN | 101407638 | A | 15 April 2009 |
| | | | | CN | 101407638 | B | 12 December 2012 |
| | | | | EP | 2176325 | A2 | 21 April 2010 |
| | | | | EP | 2176325 | B1 | 08 December 2021 |
| | | | | JP | 2010-540685 | A | 24 December 2010 |
| | | | | KR | 10-1529349 | B1 | 17 June 2015 |
| | | | | US | 2010-0209379 | A1 | 19 August 2010 |
| | | | | US | 8658146 | B2 | 25 February 2014 |
| | | | | WO | 2009-040106 | A2 | 02 April 2009 |
| | | | | WO | 2009-040106 | A3 | 21 October 2010 |
| EP | 1358894 | A1 | 05 November 2003 | None | | | |
| KR | 10-2021-0058928 | A | 24 May 2021 | CN | 112714770 | A | 27 April 2021 |
| | | | | CN | 112714770 | B | 28 March 2023 |
| | | | | JP | 7064614 | B2 | 10 May 2022 |
| | | | | KR | 10-2562511 | B1 | 03 August 2023 |
| | | | | WO | 2020-059871 | A1 | 26 March 2020 |
| KR | 10-2022-0082510 | A | 17 June 2022 | None | | | |
| US | 2010-0035757 | A1 | 11 February 2010 | CN | 101036801 | A | 19 September 2007 |
| | | | | CN | 101036801 | B | 06 August 2014 |
| | | | | EP | 1957193 | A2 | 20 August 2008 |
| | | | | EP | 1957193 | B1 | 03 October 2012 |
| | | | | JP | 2009-515691 | A | 16 April 2009 |
| | | | | JP | 2013-163818 | A | 22 August 2013 |
| | | | | KR | 10-1407781 | B1 | 20 June 2014 |
| | | | | KR | 10-2008-0077630 | A | 25 August 2008 |
| | | | | US | 8653320 | B2 | 18 February 2014 |
| | | | | WO | 2007-057203 | A2 | 24 May 2007 |
| | | | | WO | 2007-057203 | A3 | 13 December 2007 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230073035

- KR 1020240073975

**Non-patent literature cited in the description**

- UV Coatings: Basics, Recent Developments and New Application. Elsevier, 2007

- **ODIAN**. Principle of Polymerization. Wiley, 1981, 203